# EUROPÄISCHE PATENTSCHRIFT

(11) **EP 0 685 219 B1**
(45) Veröffentlichungstag und Bekanntmachung des Hinweises auf die Patenterteilung: **06.06.2001**
(21) Anmeldenummer: 95102854.7
(22) Anmeldetag: 01.03.1995
(51) Int. Cl.: A61K 7/00, A61K 7/09, A61K 7/06, A61K 7/50

(54) **Haarbehandlungsmittel und Verfahren zur Anwendung**
Hair treatment agent and process for its use
Agent de soin capillaire et procédé d'utilisation

(30) Priorität: 03.06.1994 DE 4419457
(43) Veröffentlichungstag der Anmeldung: 06.12.1995
(73) Patentinhaber: Wella Aktiengesellschaft, 64274 Darmstadt (DE)
(72) Erfinder: Kischka, Karl-Heinz, Dr., D-64293 Darmstadt (DE); Flemming, Ernst, D-63150 Heusenstamm (DE)

(56) Entgegenhaltungen:
- EP-A- 0 636 357
- WO-A-94/02115
- DE-A- 4 131 992
- US-A- 4 832 872

## Beschreibung

Gegenstand der vorliegenden Erfindung ist ein Mittel zur Behandlung der Haare auf wäßrig-alkoholischer Basis, welches frei von kationischen Tensiden ist, einen pH-Wert von 2,5 bis 6,9 aufweist und eine Kombination aus mindestens einem filmbildenden kationischen, anionischen und/oder amphoteren Polymer mit einem amphoteren Tensid, einem C2- bis C4-Alkohol und einer organischen Säure enthält sowie Verfahren zur Anwendung des Mittels.

Die Haare werden durch Einwirkungen verschiedener Art in ihrem physikalischen, chemischen und morphologischen Eigenschaften negativ beeinflußt. So wird das Haar durch kosmetische Behandlungen, wie wiederholtes Bleichen, Dauerwellen und Färben, aber auch durch häufiges Waschen der Haare mit entfettenden Tensiden, durch Klimaeinflüsse wie Feuchte- und Temperaturunterschiede oder die intensive Einwirkung von Sonnenlicht sowie durch mechanische Behandlung wie Bürsten, Kämmen und Frottieren, insbesondere im Bereich der Haarspitzen, stark strapaziert und geschädigt, während das Haar am Haaransatz eine gesunde ungeschädigte Struktur besitzt. Die meist unterschiedliche Haarstruktur zwischen Haaransatz und den Haarspitzen stellt bei der Dauerwellbehandlung der Haare ein großes Problem dar. Wird das Dauerwellmittel in seiner Wellwirksamkeit den strapazierten und geschädigten Haarspitzen angepaßt, so erhält man eine nur ungenügende Dauerverformung des Haares am Haaransatz. Paßt man dagegen das Dauerwellmittel in seiner Wellwirksamkeit der gesunden, ungeschädigten Struktur des Haares am Haaransatz an, so kann das Haar an den strapazierten Haarspitzen so sehr geschädigt werden, daß es zu einer stark eingeschränkten Lockigkeit bis hin zum Abbrechen der Haare kommen kann.

Bei der Dauerwellbehandlung der Haare mit keratinreduzierenden Verformungsmitteln wird im allgemeinen so verfahren, daß man die zuvor gewaschenen und mit einem Handtuch abfrottierten Haare mit einem Teil des Verformungsmittels durchfeuchtet, sodann in Strähnen aufteilt, diese Strähnen einzeln auf Dauerwellwickler wickelt und anschließend mit dem restlichen Verformungsmittel befeuchtet. Nach Beendigung des Verformungsvorganges werden die Haare mit Wasser gespült, sodann oxidativ fixiert, anschließend die Wickler entfernt, die Haare erneut mit Wasser gespült und gegebenenfalls mit einem Kurpräparat nachbehandelt.

Diese Verfahrensweise besitzt jedoch einige Nachteile. So kann eine derartige Verfahrensweise zu Schädigungen an den Händen des die Verformungsbehandlung ausführenden Friseurs führen (zum Beispiel Allergien oder andere Hauterkrankungen), da die Hände über die gesamte, für das Wickeln erforderliche, etwa 20 Minuten betragende Zeitspanne mit dem Verformungsmittel in Kontakt kommen. Außerdem ist das vorstehend beschriebene Verfahren nicht sehr haarschonend, da die durch das Vorfeuchten der Haare mit dem Verformungsmittel eintretende Haarerweichung beim Wickelvorgang sehr leicht zu einer Überdehnung des Haares und als Folge hiervon zu Haarbruch und Haarausfall führen kann. Zudem sind die Haare nach einer Dauerwellverformungsbehandlung meist stark strapaziert, so daß eine Nachbehandlung mit einem Haarkurmittel erforderlich ist, um dem Haar wieder einen natürlichen Griff und Glanz zu verleihen. Aus der US-Patentschrift 4,832,872 ist ein Haarbehandlungsmittel bekannt, bestehend aus einem kationischen Polymer, einem amphoteren Tensid, Ethylalkohol, einer organischen Säure und einem kationaktiven Tensid. Es bestand daher die Aufgabe, ein Mittel zur Behandlung der Haare zur Verfügung zu stellen, welches das Haar, insbesondere die Haarspitzen, während des Dauerwellvorganges schützt und so eine gleichmäßige Verformung der Haare ermöglicht. Zudem soll das Mittel gute glanzgebende Eigenschaften besitzen ohne das Haar zu belasten.
Es wurde nunmehr gefunden, daß durch ein Haarbehandlungsmittel auf wäßrig-alkoholischer Basis, dadurch gekennzeichnet, daß es

| | | |
|---|---|---|
| (A) | 0,1 bis 10 | Gewichtsprozent mindestens eines filmbildenden kationischen, anionischen und/ oder amphoteren Polymers, |
| (B) | 0,1 bis 9 | Gewichtsprozent mindestens eines amphoteren Tensids, |
| (C) | 0,1 bis 40 | Gewichtsprozent mindestens eines C2- bis C4-Alkohols und |
| (D) | 0,01 bis 2,0 | Gewichtsprozent mindestens einer organischen Säure |

enthält und frei von kationischen Tensiden ist und einen pH-Wert von 2,5 bis 6,9 aufweist, die gestellte Aufgabe in hervorragender Weise erfüllt wird.

Das erfindungsgemäße Mittel enthält die Komponente (A) bevorzugt in einer Menge von 0,1 bis 4 Gewichtsprozent.

Beispiele für als Komponente (A) geeignete filmbildende kationische Polymere sind quaternisierte Vinylpyrrolidon/ Dimethylaminoethylmethacrylat-Copolymere, Dimethyldiallylammoniumchlorid-Polymere, quaternäre Acrylamid /Dimethyldiallylammoniumchlorid-Copolymere, quaternäre Methylvinylimidazolinium/Vinylpyrrolidon-Copolymere oder kationische Chitosanderivate.

Geeignete quaternisierte Vinylpyrrolidon/Dimethylaminoethylmethacrylat-Copolymere werden beispielsweise von der Firma Gaf Co., New York, USA in Form einer 20prozentigen wäßrigen Lösung unter der Handelsbezeichnung Gafquat® 755 N und in Form einer 50prozentigen Lösung in Ethanol unter der Handelsbezeichnung Gafquat® 734 vertrieben. Als Komponente (A) des erfindungsgemäßen Mittels geeignete Dimethyldiallyamoniumchlorid-Polymere werden beispielsweise von der Firma Merck & Co. Inc., New Jersey, USA, in Form einer 40prozentigen wäßrigen Lösung unter der Handelsbezeichnung Merquat® 100 und geeignete quaternäre Acrylamid/Dimethyldiallylammoniumchlorid-Copolymere werden von der gleichen Firma in Form einer 8prozentigen wäßrigen Lösung unter der Handelsbezeichnung Merquat® 550 vertrieben.

Als Komponente (A) geeignete quaternäre Methylvinylimidazolinium/Vinylpyrrolidon-Copolymere werden unter der Handelsbezeichnung Luviquat® FC 905 in Form einer 40prozentigen wäßrigen Lösung von der Firma BASF AG, Ludwigshafen, BRD vertrieben.

Als Komponente (A) geeignete filmbildende anionische Polymere sind beispielsweise Schellack oder Vinylacetat/Crotonsäure-Copolymere, die zum Beispiel von der Firma Hoechst AG, Frankfurt, BRD in Form einer 60prozentigen Lösung in wäßrigem Isopropanol unter der Handelsbezeichnung Aristoflex® A und von der Firma Delft-National GmbH, Frankfurt, BRD unter der Handelsbezeichnung Resyn® 28-1310 vertrieben werden.

Ein als Komponente (A) geeignetes filmbildendes amphoteres Polymer ist'beispielsweise das Octylacrylamid/Acrylat/Butylaminoäthyl/Methacrylat-Copolymer, das von der National Starch and Chem. Co., New Jersey, USA unter der Handelsbezeichnung Amphomer® vertrieben wird.

Von den als Komponente (A) geeigneten filmbildenden kationischen, anionischen und amphoteren Polymeren sind die filmbildenden kationischen Polymere bevorzugt in dem erfindungsgemäßen Mittel enthalten.

Das erfindungsgemäße Haarbehandlungsmittel enthält als Komponente (B) bevorzugt 0,1 bis 4 Gewichtsprozent mindestens eines amphoteren Tensids.

Beispiele für als Komponente (B) geeignete amphotere Tenside sind Alkylbetaine, Alkylaminobetaine, Fettsäureamidoalkylbetaine und Fettsäureamidoalkylsulfobetaine.

Die Komponente (B) des erfindungsgemäßen Mittels ist bevorzugt ausgewählt aus Fettsäureamidoalkylbetain und Fettsäureamidoalkylsulfobetain oder deren Gemisch.

Besonders bevorzugt enthält das erfindungsgemäße Mittel als Komponente (B) Kokosfettsäureamidopropylbetain, das beispielsweise von der Firma Goldschmidt, Essen, BRD unter der Handelsbezeichnung Tego Betain® L7 in Form einer 30prozentigen wäßrigen Lösung vertrieben wird, und/oder 3-(3-Kokosfettsäureamidopropyl)dimethylammonium-2-hydroxypropan-sulfonat, das beispielsweise in Form einer 50prozentigen wäßrigen Lösung unter der Handelsbezeichnung Rewoteric® AM-CAS von der Firma Rewo Chemische Werke GmbH, Steinau, BRD, unter der Handelsbezeichnung Mirataine® CBS von der Firma Miranol Chem. Co. Inc., New Jersey, USA und unter der Handelsbezeichnung Schercotaine® SCAB-A von der Firma Scher Chem. Inc., New Jersey, USA vertrieben wird.

Das erfindungsgemäße Mittel enthält die Komponente (C) bevorzugt in einer Menge von 5 bis 25 Gewichtsprozent. Die als Komponente (C) geeigneten Alkohole können eine geradkettige oder verzweigte C2- bis C4-Alkylkette aufweisen. Die Komponente (C) des erfindungsgemäßen Mittels ist bevorzugt ausgewählt aus Ethanol und Isopropanol oder deren Gemisch.

Das erfindungsgemäße Mittel enthält bevorzugt 0,01 bis 1 Gewichtsprozent der Komponente (D).

Das Mittel zur Behandlung der Haare gemäß der vorliegenden Erfindung enthält als Komponente (D) mindestens eine organische Säure, welche bevorzugt ausgewählt ist aus Zitronensäure, Weinsäure, Glyoxylsäure, Milchsäure und Ameisensäure.

In einer bevorzugten Ausführungsform enthält das erfindungsgemäße Mittel zusätzlich zu den Komponenten (A) bis (D) 0,1 bis 10 Gewichtsprozent, vorzugsweise 0,1 bis 4 Gewichtsprozent, Polyvinylpyrrolidon und/oder Chitosan, wobei ein Zusatz von Polyvinylpyrrolidon besonders bevorzugt ist.

Geeignete Polyvinylpyrrolidone werden beispielsweise unter der Handelsbezeichnung Luviskol® K 90 von der Firma BASF, Ludwigshafen, BRD und unter der Handelsbezeichnung PVP/K® von der Firma ISP, Surrey, Großbritannien vertrieben.

Das erfindungsgemäße Mittel kann zusätzlich zu der Kombination aus den Komponenten (A) bis (D) 0,1 bis 5 Gewichtsprozent mindestens eines nicht-ionischen Tensids enthalten.

Beispiele für nicht-ionische Tenside, die zusätzlich in dem erfindungsgemäßen Mittel enthalten sein können, sind die Alkylpolyglucoside, wie zum Beispiel Laurylpolyglucose, die in Form einer 50prozentigen wäßrigen Lösung unter der Handelsbezeichnung Plantaren® 1200 CS/UP von der Firma Henkel KGaA, Düsseldorf, BRD vertrieben wird.

Das erfindungsgemäße Mittel weist einen pH-Wert von 2,5 bis 6,9, bevorzugt von 2,5 bis 4,5,auf.

Das Haarbehandlungsmittel gemäß der vorliegenden Erfindung kann zusätzlich alle diejenigen Bestandteile enthalten, die in Haarbehandlungsmitteln üblicherweise eingesetzt werden, insbesondere Schaumsynergisten; Schaumstabilisatoren,Sequestriermittel; Naturstoffe; Pigmente; Parfümöle in einer Menge von 0,05 bis 3,0 Gewichtsprozent; Trübungsmittel, wie zum Beispiel Ethylenglykoldistearat, in einer Menge von etwa 0,05 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; Puffersubstanzen, wie beispielsweise Natriumcitrat oder Natriumphosphat, in einer Menge von 0,1 bis 1,0 Gewichtsprozent; sowie Farbstoffe, wie zum Beispiel Fluorescein-Natriumsalz, Gelb ZN3 (C.I. 47 055), in einer Menge von 0,1 bis 1,0 Gewichtsprozent; weiterhin haarpflegende Zusätze, wie zum Beispiel Lanolinderivate, Keratinhydrolysat, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel, Lichtschutzmittel, Antioxidantien, Komplexbildner, Antischuppenwirkstoffe, kosmetische Öle und Wachse sowie Konservierungsstoffe, soweit solche Zusätze nützlich und zweckmäßig erscheinen und mit den Bestandteilen des erfindungsgemäßen Mittels verträglich sind.

Das erfindungsgemäße Mittel weist bevorzugt einen Wassergehalt von 70 bis 90 Gewichtsprozent auf.

Das erfindungsgemäße Haarbehandlungsmittel liegt in Form einer Zubereitung vor, die nach der Anwendung nicht ausgespült wird und kann als Haarstrukturregenerator vor der Wasserwelle, der Fönwelle, der Heißwelle oder vor einer Dauerwellbehandlung sowie als Lockenauffrischer für dauer- oder naturgewelltes Haar angewandt werden.

Das erfindungsgemäße Mittel weist hervorragende konditionierende Eigenschaften auf. Das erfindungsgemäße Haarbehandlungsmittel verleiht dem Haar einen schönen Glanz ohne die Haltbarkeit der Frisur zu beeinträchtigen. Die Kämmbarkeit und der Griff des Haares werden durch die Verwendung des erfindungsgemäßen Mittels deutlich verbessert.

Das erfindungsgemäße Mittel wird bevorzugt als Dauerwellvorbehandlungsmittel, das heißt zur Behandlung des Haares vor dem Wickeln auf Dauerwellwickler, verwendet.

Als Dauerwellvorbehandlungsmittel verwendet, bewirkt das erfindungsgemäße Mittel eine gleichmäßige Verformung der Haare während des Dauerwellvorgangs. Die empfindlichen Haarspitzen und strukturgeschädigtes Haar werden durch die Verwendung des erfindungsgemäßen Mittels als Dauerwellvorbehandlungsmittel während des Dauerwellvorganges geschützt, so daß eine schonende Dauerverformung des Haares ermöglicht wird.

Insbesondere strukturgeschädigtes Haar weist poröse Bereiche überwiegend in der Schuppenschicht, aber auch im Faserstamm, auf. Diese porösen Bereiche werden durch das erfindungsgemäße Mittel ausgeglichen, das bevorzugt die geschädigten Bereiche des Haares nachweisbar umhüllt, so daß die haarschädigende Wirkung der Dauerwellösung vermindert werden kann.

Zudem wird insbesondere durch die Komponente (D) des erfindungsgemäßen Mittels die Schließung der Schuppenschicht (Cuticula) des Haares gefördert, so daß die Diffusion der wellaktiven Bestandteile der Dauerwelllösung in das Haar verzögert wird.

Durch die Verwendung des erfindungsgemäßen Haarbehandlungsmittels als Dauerwellvorbehandlungsmittel wird zudem das Wickeln der Haare auf die Dauerwellwickler erheblich erleichtert. Darüber hinaus bewirkt das erfindungsgemäße Mittel, wenn es vor einer Dauerwellbehandlung angewendet wird, eine deutliche Verbesserung der Sprungkraft und der Elastizität des dauergewellten Haares.

Das erfindungsgemäße Mittel liegt vorzugsweise in Form einer wäßrig-alkoholischen Lösung vor und kann unter Verwendung eines Treibmittels oder mit Hilfe einer mechanisch betriebenen Sprühvorrichtung oder Schaumerzeugungsvorrichtung versprüht oder als Schaum abgegeben werden.

Wenn das erfindungsgemäße Mittel mit Hilfe eines Treibmittels versprüht wird, so enthält es bevorzugt 3 bis 20 Gewichtsprozent des Treibmittels und wird in einen Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise N2, N20 und CO₂, sowie Gemische der vorstehend genannten Treibmittel geeignet.

Unter mechanischen Sprühvorrichtungen oder Schaumerzeugungsvorrichtungen sind solche Vorrichtungen zu verstehen, welche das Versprühen oder Aufschäumen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Als geeignete mechanische Schaumerzeugungsvorrichtung kann beispielsweise der in der EP-B 0 460 154 beschriebene Aufsatz mit einer Schaumerzeugungsvorrichtung auf einem elastischen Behälter verwendet werden.

Die vorliegende Erfindung betrifft weiterhin ein Verfahren zur dauerhaften Verformung von Haaren, bei dem man das Haar vor dem Wickeln auf Dauerwellwickler mit einem Dauerwellvorbehandlungsmittel behandelt,das Haar trocknet, erneut mit Wasser anfeuchtet, auf Wickler wickelt, sodann mit einem Haardauerverformungsmittel behandelt, nach einer für die dauerhafte Verformung des Haares ausreichenden Einwirkungszeit mit Wasser spült, sodann oxidativ nachbehandelt, mit Wasser spült, anschließend zur Frisur legt und sodann trocknet, welches dadurch gekennzeichnet ist, daß als Dauerwellvorbehandlungsmittel das vorstehend beschriebene erfindungsgemäße Haarbehandlungsmittel verwendet wird.

Bei dem erfindungsgemäßen Verfahren wird das Haar zunächst mit einem Shampoo gewaschen und danach mit Wasser gespült. Anschließend wird eine für die Dauerwellvorbehandlung ausreichende Menge, vorzugsweise etwa 10 bis 20 g, des erfindungsgemäßen Haarbehandlungsmittels auf das handtuchtrockene Haar, vorzugsweise auf die Haarspitzen, aufgetragen. Nach einer ausreichenden Einwirkzeit, welche in Abhängigkeit von der Haarbeschaffenheit 5 bis 15 Minuten beträgt, wird das Haar getrocknet. Sodann wird das Haar mit Wasser angefeuchtet, ohne jedoch das Dauerwellvorbehandlungsmittel aus dem Haar auszuspülen. Das Haar wird in Strähnen aufgeteilt und auf Dauerwellwickler gewickelt. Der Durchmesser der Wickler beträgt hierbei etwa 5 bis 35 Millimeter. Anschließend wird das Haar mit einer für die dauerhafte Haarverformung ausreichenden Menge, vorzugsweise etwa 60 bis 90 g, eines Haardauerverformungsmittels behandelt.

Bei dem Haardauerverformungsmittel handelt es sich insbesondere um eine wäßrige, alkalisch (pH = 7 bis 10) eingestellte Zubereitung, welche eine keratinreduzierende Mercaptoverbindung, wie zum Beispiel Cystein, Cysteamin, N-Acetyl-L-cystein, Mercaptocarbonsäure, beispielsweise Thioglykolsäure oder Thiomilchsäure, oder Salze von Mercaptocarbonsäuren, wie zum Beispiel Ammonium- und Guanidinsalze der Thioglykolsäure oder Thiomilchsäure, in einer Konzentration von etwa 2 bis 12 Gewichtsprozent enthält.

Die erforderliche Alkalität wird hierbei durch die Zugabe von Ammoniak, organischen Aminen, Ammonium- und Alkalicarbonaten oder -hydrogencarbonaten eingestellt. Es kommt aber auch ein neutral oder sauer (pH = 4,5 bis 7) eingestelltes Haarverformungsmittel in Betracht, das im wäßrigen Medium einen wirksamen Gehalt an Sulfiten oder Mercaptocarbonsäureestern aufweist.

Im ersteren Fall werden vorzugsweise Natrium- oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als S02) verwendet. Im letzteren Fall kommen insbesondere Thioglykolsäuremonoglykolester oder -glycerinester in einer Konzentration von etwa 5 bis 50 Gewichtsprozent (entsprechend einem Gehalt an freier Thioglykolsäure von 2 bis 16 Gewichtsprozent) zur Anwendung.

Das Mittel zur dauerhaften Haarverformung kann auch ein Gemisch der vorstehend genannten keratinreduzierenden Verbindungen enthalten.

Nach einer für die dauerhafte Verformung des Haares ausreichenden Einwirkungszeit, welche, je nach Haarbeschaffenheit, dem pH-Wert und der Verformungswirksamkeit des Verformungsmittels sowie in Abhängigkeit von der Anwendungstemperatur, etwa 10 bis 30 Minuten beträgt, wird das Haar mit Wasser gespült und anschließend oxidativ nachbehandelt ("fixiert"). Das Nachbehandlungsmittel wird, je nach Haarfülle, in einer Menge von etwa 50 bis 100 g verwendet.

Für die oxidative Nachbehandlung kann jedes beliebige, bisher für eine derartige Behandlung verwendete Nachbehandlungsmittel verwendet werden. Beispiele für in einem derartigen Nachbehandlungsmittel verwendbare Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich. Üblicherweise liegt das Oxidationsmittel in dem wäßrigen Nachbehandlungsmittel in einer Konzentration von etwa 0,5 bis 10 Gewichtsprozent vor.

Sowohl das Mittel zur dauerhaften Haarverformung als auch das Mittel zur oxidativen Nachbehandlung kann in Form einer wäßrigen Lösung oder Emulsion sowie in verdickter Form auf wäßriger Basis, insbesondere als Creme, Gel oder Paste, vorliegen. Es ist ebenfalls möglich, dieses Mittel unter Druck in Aerosoldosen abzufüllen und daraus als Schaum zu entnehmen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet.

Das vorstehend beschriebene erfindungsgemäße Verfahren zur dauerhaften Haarverformung ermöglicht eine schonende und gleichmäßige Umformung vom Haaransatz bis zu den Haarspitzen. Das Haar zeigt eine hervorragende Naß- und Trockenkämmbarkeit, einen angenehmen Griff und einen ansprechenden Glanz im getrockneten Zustand, sowie eine lockere, sprunghafte und gleichmäßige Dauerwellung, insbesondere im Bereich der Haarspitzen.

Ein weiterer Gegenstand der vorliegenden Anmeldung ist ein, auch ohne nachfolgende Dauerwellbehandlung anzuwendendes, Verfahren zur Behandlung von Haaren, bei dem man nach der Haarwäsche das erfindungsgemäße Mittel, je nach Haarfülle, in einer Menge von 10 bis 20 g im handtuchtrockenen Haar verteilt. Nach einer ausreichenden Einwirkungszeit, welche in Abhängigkeit von der Haarbeschaffenheit 5 bis 15 Minuten beträgt, wird das Haar getrocknet. Anschließend wird das Haar mit Wasser angefeuchtet, gegebenenfalls zur Frisur gelegt und sodann getrocknet.

Gegenstand der vorliegenden Anmeldung ist weiterhin ein Verfahren zur Behandlung von natur- oder dauergewellten Haaren, das zur Lockenauffrischung dient, bei dem man im trockenen oder mit Wasser angefeuchteten natur- oder dauergewellten Haar, je nach
Haarfülle, 10 bis 20 g des erfindungsgemäßen Mittels verteilt, wobei das erfindungsgemäße Mittel vorzugsweise auf das Haar gesprüht wird, sodann die Haare vorzugsweise mit den Händen, zur Frisur formt und anschließend trocknet.

Die mit dem erfindungsgemäßen Mittel behandelten Haare weisen eine hervorragende Naß- und Trockenkämmbarkeit, einen angenehmen Griff und einen ansprechenden Glanz im getrockneten Zustand sowie einen sehr guten Halt der Frisur auf.

Folgende Beispiele sollen den Gegenstand der Erfindung näher erläutern:

### Beispiele

### Beispiel 1: Haarbehandlungsmittel

| | |
|---|---|
| 0,600 g | Polyvinylpyrrolidon |
| 0,200 g | Vinylpyrrolidon/Dimethylaminoethylmethacrylat-Copolymer (Gafquat® 755 N der Firma Gaf Co./USA) |
| 0,400 g | 3-(3-Kokosfettsäureamidopropyl)dimethylammonium-2-hydroxypropansulfonat |
| 0,010 g | Keratinhydrolysat |
| 0,100 g | Glyoxylsäure |
| 12,000 g | Ethanol |
| 86,690 g | Wasser |
| 100,000 g | |

### Beispiel 2: Haarbehandlungsmittel

| | |
|---|---|
| 0,600g | Polyvinylpyrrolidon |
| 0,200g | Methylvinylimidazolinium/Vinylpyrrolidon-Copolymer (Luviquat® FC 905 der Firma BASF AG, BRD) |
| 0,450g | Kokosfettsäureamidopropylbetain |
| 0,020g | Keratinhydrolysat |
| 0,200g | Zitronensäure |
| 10,000g | Isopropanol |
| 88,530g | Wasser |
| 100,000 g | |

### Beispiel 3: Haarbehandlungsmittel

| | |
|---|---|
| 1,280 g | Methylvinylimidazolinium/Vinylpyrrolidon-Copolymer (Luviquat® FC 905 der Firma BASF AG, BRD) |
| 0,300 g | Kokosfettsäureamidopropylbetain |
| 0,500 g | Kamillenblütenextrakt (Extrapon® Kamille Spezial 2/033021 der Firma Dragoco, Holzminden, BRD) |
| 0,160 g | Keratinhydrolysat |
| 0,250 g | Zitronensäure |
| 15,000 g | Ethanol |
| 82,510 g | Wasser |
| 100,000 g | |

### Beispiel 4: Haarbehandlungsmittel

| | |
|---|---|
| 0,640 g | Dimethyldiallylammoniumchlorid Polymer (Merquat® 100 der Firma Gaf Co./USA) |
| 0,300 g | Kokosfettsäureamidopropylbetain |
| 0,250 g | Zitronensäure |
| 0,500 g | Kamillenblütenextrakt (Extrapon® Kamille Spezial 2/033021 der Firma Dragoco/BRD) |
| 15,000 g | Ethanol |
| 83,310 g | Wasser |
| 100,000 g | |

### Beispiel 5: Haarbehandlungsmittel

| | |
|---|---|
| 0,144 g | Acrylamid/Dimethyldiallylammoniumchlorid-Copolymer (Merquat® 550 der Firma Gaf Co./USA) |
| 0,300 g | Kokosfettsäureamidopropylbetain |
| 0,160 g | Keratinhydrolysat |
| 0,250 g | Zitronensäure |
| 0,500 g | Kamillenblütenextrakt (Extrapon® Kamille-Spezial 2/033021 der Firma Dragoco/BRD) |
| 15,00 g | Ethanol |
| 83,646 g | Wasser |
| 100,000 g | |

### Beispiel 6: Haarbehandlungsmittel

| | |
|---|---|
| 0,450 g | Vinylpyrrolidon/Dimethylaminoethylmethacrylat-Copolymer (Gafquat®734 der Firma Gaf Co./USA) |
| 0,300 g | Kokosfettsäureamidopropylbetain |
| 0,160 g | Keratinhydrolysat |
| 0,500 g | Kamillenblütenextrakt (Extrapon® Kamille Spezial 2/033021 der Firma Dragoco/BRD) |
| 0,250 g | Zitronensäure |
| 15,450 g | Ethanol |
| 82,890 g | Wasser |
| 100,000 g | |

### Beispiel 7: Haarbehandlungsmittel

| | |
|---|---|
| 0,280 g | Vinylpyrrolidon/Dimethylaminoethyl methacrylat-Copolymer (Gafquat® 755 N der Firma Gaf Co./USA) |
| 0,300 g | Kokosfettsäureamidopropylbetain |
| 0,500 g | Kamillenblütenextrakt (Extrapop Kamille Spezial 2/033021 der Firma Dragoco/BRD) |
| 0,250 g | Zitronensäure |
| 15,000 g | Ethanol |
| 83,670 g | Wasser |
| 100,000 g | |

### Beispiel 8: Haarbehandlungsmittel

| | |
|---|---|
| 0,600 g | Vinylacetat/Crotonsäure-Copolymer (Aristoflex® A der Firma Hoechst AG, BRD) |
| 0,800 g | Laurylpolyglucose |
| 0,150 g | Kokosfettsäureamidopropylbetain |
| 0,120 g | Keratinhydrolysat |
| 0,100 g | Milchsäure |
| 25,000 g | Ethanol |
| 73,230 g | Wasser |
| 100,000 g | |

Alle Prozentangaben in dieser Anwendung stellen, soweit nicht anders angegeben, Gewichtsprozente dar.

## Patentansprüche

1. Mittel zur Behandlung der Haare auf wäßrig-alkoholischer Basis, dadurch gekennzeichnet, daß es
| | | |
|---|---|---|
| (A) | 0,1 bis 10 | Gewichtsprozent mindestens eines filmbildenden kationischen,anionischen und/oder amphoteren Polymers, |
| (B) | 0,1 bis 9 | Gewichtsprozent mindestens eines amphoteren Tensids, |
| (C) | 0,1 bis 40 | Gewichtsprozent mindestens eines C2- bis C4-Alkohols und |
| | | |
| (D) | 0,01 bis 2,0 | Gewichtsprozent mindestens einer organischen Säure |
enthält und frei von kationischen Tensiden ist und einen pH-Wert von 2,5 bis 6,9 aufweist.

2. Mittel nach Anspruch 1, dadurch gekennzeichnet, daß es 0,1 bis 4 Gewichtsprozent der Komponente (A) enthält.

3. Mittel nach Anspruch 1 oder 2, dadurch gekennzeichnet, daß die Komponente (B) aus gewählt aus Fettsäureamidoalkylbetain und Fettsäureamidoalkylsulfobetain oder deren Gemisch.

4. Mittel nach einem der Ansprüche 1 bis 3, dadurch gekennzeichnet, das es 0,1 bis 4 Gewichtsprozent der Komponente (B) enthält.

5. Mittel nach einem der Ansprüche 1 bis 4, dadurch gekennzeichnet, daß es 5 bis 25 Gewichtsprozent der Komponente (C) enthält.

6. Mittel nach einem der Ansprüche 1 bis 5, dadurch gekennzeichnet, daß die Komponente (C) ausgewählt ist aus Ethanol und Isopro panol oder deren Gemisch.

7. Mittel nach einem der Ansprüche 1 bis 6, dadurch gekennzeichnet, daß es 0,01 bis 1 Gewichtsprozent der Komponente (D) enthält.

8. Mittel nach einem der Ansprüche 1 bis 7, dadurch gekennzeichnet, daß die organische Säure der Komponente (D) ausgewählt ist aus Zitronensäure, Weinsäure, Glyoxylsäure, Milchsäure und Ameisensäure.

9. Mittel nach einem der Ansprüche 1 bis 8, dadurch gekennzeichnet, daß es zusätzlich 0,1 bis 10 Gewichtsprozent Polyvinylpyrrolidon und/oder Chitosan enthält.

10. Mittel nach einem der Ansprüche 1 bis 9, dadurch gekennzeichnet, daß es zusätzlich 0,1 bis 5 Gewichtsprozent mindestens eines nicht-ionischen Tensids enthält.

11. Verfahren zur dauerhaften Verformung von Haaren, bei dem man das Haar vor dem Wickeln auf Dauerwellwickler mit einem Dauerwellvorbehandlungsmittel behandelt, das Haar trocknet, erneut mit Wasser anfeuchtet, auf Wickler wickelt, sodann mit einem Haardauerverformungsmittel behandelt, nach einer für die dauerhafte Verformung des Haares ausreichenden Einwirkungszeit mit Wasser spült, sodann oxidativ nachbehandelt, mit Wasser spült, anschließend zur Frisur legt und sodann trocknet, dadurch gekennzeichnet, das als Dauerwellvorbehandlungsmittel ein Mittel nach einem der Ansprüche 1 bis 10 verwendet wird.

12. Verfahren nach Anspruch 11, dadurch gekennzeichnet, das man das Dauerwellvorbehandlungsmittel in einer Menge von 10 bis 20 g anwendet.

13. Verfahren zur Behandlung von Haaren, dadurch gekennzeichnet, daß man nach der Haarwäsche das handtuchtrockene Haar mit einem Mittel nach einem der Ansprüche 1 bis 10 in einer Menge von 10 bis 20 g behandelt, das Haar trocknet, erneut mit Wasser anfeuchtet und sodann trocknet.

14. Verfahren zur Behandlung von natur- oder dauergewellten Haaren, dadurch gekennzeichnet, das man im trockenen oder mit Wasser angefeuchteten natur- oder dauergewellten Haar 10 bis 20 g eines Mittels nach einem der Ansprüche 1 bis 10 verteilt, sodann die Haare zur Frisur formt und anschließend trocknet.

## Claims

1. Composition for the treatment of hair on an aqueous-alcoholic basis, characterized in that it comprises
| | | |
|---|---|---|
| (A) | 0.1 to 10 | per cent by weight of at least one film-forming cationic, anionic and/or amphoteric-polymer, |
| (B) | 0.1 to 9 | per cent by weight of at least one amphoteric surfactant, |
| (C) | 0.1 to 40 | per cent by weight of at least one C₂-C₄-alcohol and |
| (D) | 0.01 to 2.0 | per cent by weight of at least one organic acid |
and is free from cationic surfactants and has a pH of from 2.5 to 6.9.

2. Composition according to Claim 1, characterized in that it comprises 0.1 to 4 per cent by weight of component (A).

3. Composition according to Claim 1 or 2, characterized in that component (B) is chosen from fatty acid amidoalkylbetaine and fatty acid amidoalkylsulphobetaine or a mixture thereof.

4. Composition according to one of Claims 1 to 3, characterized in that it comprises 0.1 to 4 per cent per weight of component (B).

5. Composition according to one of Claims 1 to 4, characterized in that it comprises 5 to 25 per cent by weight of component (C).

6. Composition according to one of Claims 1 to 5, characterized in that component (C) is chosen from ethanol and isopropanol or a mixture thereof.

7. Composition according to one of Claims 1 to 6, characterized in that it comprises 0.01 to 1 per cent by weight of component (D).

8. Composition according to one of Claims 1 to 7, characterized in that the organic acid of component (D) is chosen from citric acid, tartaric acid, glyoxylic acid, lactic acid and formic acid.

9. Composition according to one of Claims 1 to 8, characterized in that it additionally comprises 0.1 to 10 per cent by weight of polyvinylpyrrolidone and/or chitosan.

10. Composition according to one of Claims 1 to 9, characterized in that it additionally comprises 0.1 to 5 per cent by weight of at least one nonionic surfactant.

11. Method of permanently shaping hair in which the hair, prior to winding on permanent wave curlers, is treated with a permanent wave pretreatment composition, the hair is dried, again dampened with water, wound onto curlers, then treated with a hair permanent shaping composition, rinsed with water after a contact time sufficient for the permanent shaping of the hair, then oxidatively after-treated, rinsed with water, then styled and then dried, characterized in that the permanent wave pretreatment composition used is a composition according to one of Claims 1 to 10.

12. Method according to Claim 11, characterized in that the permanent wave pretreatment composition is applied in an amount of from 10 to 20 g.

13. Method of treating hair, characterized in that after the washing of hair, the towel-dried hair is treated with a composition according to one of Claims 1 to 10 in an amount of from 10 to 20 g, the hair is dried, dampened again with water and then dried.

14. Method of treating naturally wavy or permanently waved hair, characterized in that 10 to 20 g of a composition according to one of Claims 1 to 10 is distributed in the dry or water-dampened naturally wavy or permanently waved hair, then the hair is styled and subsequently dried.

## Revendications

1. Composition pour le traitement des cheveux à base aqueuse/alcoolique, caractérisée en ce qu'elle comprend
| | | |
|---|---|---|
| (A) | de 0,1 à 10 | pour cent en poids d'au moins un polymère filmogène cationique, anionique et/ou amphotère, |
| (B) | de 0,1 à 9 | pour cent en poids d'au moins un tensioactif amphotère, |
| (C) | de 0,1 à 40 | pour cent en poids d'au moins un alcool en C2 à C4 et |
| (D) | de 0,01 à 2,0 | pour cent en poids d'au moins un acide organique, |
et est exempte de tensioactifs cationiques et présente un pH de 2,5 à 6,9.

2. Composition selon la revendication 1, caractérisée en ce qu'elle comprend de 0,1 à 4 pour cent en poids du composant (A).

3. Composition selon la revendication 1 ou 2, caractérisée en ce que le composant (B) est choisi parmi une amidoalkylbétaïne d'acide gras et une amidoalkylsulfobétaïne d'acide gras ou leur mélange.

4. Composition selon l'une quelconque des revendications 1 à 3, caractérisée en ce qu'elle comprend de 0,1 à 4 pour cent en poids du composant (B).

5. Composition selon l'une quelconque des revendications 1 à 4, caractérisée en ce qu'elle comprend de 5 à 25 pour cent en poids du composant (C).

6. Composition selon l'une quelconque des revendications 1 à 5, caractérisée en ce que le composant (C) est choisi parmi l'éthanol et l'isopropanol ou leur mélange.

7. Composition selon l'une quelconque des revendications 1 à 6, caractérisée en ce qu'elle comprend de 0,01 à 1 pour cent en poids du composant (D).

8. Composition selon l'une quelconque des revendications 1 à 7, caractérisée en ce que l'acide organique du composant (D) est choisi parmi l'acide citrique, l'acide tartrique, l'acide glyoxylique, l'acide lactique et l'acide formique.

9. Composition selon l'une quelconque des revendications 1 à 8, caractérisée en ce qu'elle comprend en outre de 0,1 à 10 pour cent en poids de polyvinylpyrrolidone et/ou de chitosane.

10. Composition selon l'une quelconque des revendications 1 à 9, caractérisée en ce qu'elle comprend en outre de 0,1 à 5 pour cent en poids d'au moins un tensioactif non ionique.

11. Procédé d'ondulation permanente des cheveux, dans lequel on traite les cheveux par une composition de prétraitement pour permanente avant l'enroulement sur des rouleaux pour permanente, on sèche les cheveux, on les humecte de nouveau avec de l'eau, on les enroule sur des rouleaux, puis on les traite par une composition d'ondulation permanente des cheveux, on rince à l'eau après une période d'action suffisante pour l'ondulation permanente des cheveux, puis on procède à un post-traitement oxydatif, on rince à l'eau, puis on coiffe et ensuite on sèche, caractérisé en ce que l'on utilise une composition selon l'une quelconque des revendications 1 à 10 en tant que composition de prétraitement pour permanente.

12. Procédé selon la revendication 11, caractérisé en ce que l'on utilise la composition de prétraitement pour permanente en une quantité de 10 à 20 g.

13. Procédé de traitement des cheveux, caractérisé en ce qu'après avoir lavé les cheveux, on traite les cheveux séchés avec une serviette par une composition selon l'une quelconque des revendications 1 à 10 en une quantité de 10 à 20 g, on sèche les cheveux, on humecte à nouveau avec de l'eau, puis on sèche.

14. Procédé de traitement de cheveux à ondulation naturelle ou permanente, caractérisé en ce que l'on répartit, sur des cheveux à ondulation naturelle ou permanente secs ou humectés avec de l'eau, de 10 à 20 g d'une composition selon l'une quelconque des revendications 1 à 10, puis on coiffe les cheveux et on les sèche ensuite.
